# EUROPEAN PATENT APPLICATION

(11) **EP 4 321 155 A1**
(43) Date of publication of application: **14.02.2024**
(21) Application number: 23189995.6
(22) Date of filing: 07.08.2023
(51) Int. Cl.: A61K 9/20, A61K 9/28, A61K 31/472, A61K 9/48

(54) **AN ORAL PHARMACEUTICAL COMPOSITION COMPRISING ROXADUSTAT**

(30) Priority: 08.08.2022 TR 202212497
(71) Applicant: SANOVEL ILAC SANAYI VE TICARET A.S., Istanbul 34460 (TR)
(72) Inventor: OZDEN, Aydan, Istanbul (TR); PEHLIVAN AKALIN, Nur, Istanbul (TR); SUNEL, Fatih, Istanbul (TR)
(74) Representative: Sevinç, Erkan

(57) **Abstract**

The present invention relates to an oral pharmaceutical composition comprising roxadustat and at least one pharmaceutically acceptable excipient wherein the composition is free of titanium oxide. The composition is prepared by a process which is simple, rapid, cost effective, time-saving and industrially convenient.

## Description

### Field of the Invention

The present invention relates to an oral pharmaceutical composition comprising roxadustat and at least one pharmaceutically acceptable excipient wherein the composition is free of titanium oxide. The composition is prepared by a process which is simple, rapid, cost effective, time-saving and industrially convenient.

### Background of the Invention

Roxadustat is an orally available hypoxia inducible factor (HIF) prolyl hydroxylase inhibitor. HIF prolyl hydroxylase inhibitors are useful for increasing the stability and/or activity of HIF, and thus are useful for treating and preventing HIF associated disorders including anemia-, ischemia- and hypoxia-related disorders.

The chemical name of roxadustat is [(4-hydroxy-1-methyl-7-phenoxyisoquinoline- 3-carbonyl) amino]acetic acid and has a chemical structure which is shown in the Formula I.

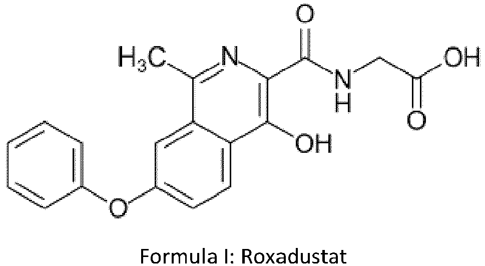

Roxadustat is available on the market in the form of a free base and is sold under trade name Evrenzo^{®} which is film coated tablets. It is an oral hypoxia-inducible factor prolyl hydroxylase (HIF-PH) inhibitor. Roxadustat has been approved for the treatment of renal anemia in patients on dialysis in September 2019 in Japan. By inhibiting HIF-PH, roxadustat suppresses the degradation of HIF-a, a subunit of the heterodimeric transcription factor HIF, leading to its accumulation and activation of the HIF pathway. Similar to activation of the HIF pathway in response to hypoxia, the production of endogenous erythropoietin is increased, and erythropoiesis is stimulated. This improves the oxygen supply to your body and may reduce your symptoms of anaemia.

Roxadustat undergoes photodecomposition after light exposure to convert to a photodegradation product.

US 20160120859A1 discloses a pharmaceutical formulation comprising Roxadustat and a pharmaceutically acceptable excipient, and an effective amount of a photostabilizing agent. Preferably, in this application document, titanium dioxide is chosen as the photostabilizing agent.

There still remains a need in the art to provide an improved an oral pharmaceutical composition comprising roxadustat having high solubility, excellent pharmacomechanic properties and accordingly a high bioavailability and a long-term stability which is also obtained by using an effective process.

### Detailed Description of the Invention

The main object of the present invention is to eliminate problems caused by active substance and bringing additional advantages to the relevant prior art. To explain in more detail, the main purpose is to provide an oral pharmaceutical composition comprising roxadustat having high physical and chemical stability and a long shelf life by the help of selection of excipients.

Another object of the present invention is to obtain an oral pharmaceutical composition comprising roxadustat providing improved dissolution profile.

Roxadustat undergoes photodecomposition after light exposure to convert to a photodegradation product. This causes stability problems. Therefore, the excipients and processes used are very important in formulations containing this molecule. Especially, to overcome photodegradation disadvantage, excipients with dye properties are used. One of them is titanium dioxide.

It is known that the moisture retention feature of titanium dioxide disrupts the body's water balance, accumulates in the body in the long term and causes DNA damage. Titanium dioxide entering the body causes accumulation in the tissues. It has a toxic and degenerative effect on the body. Inhalation increases the risk of inflammation and asthma. To eliminate all these disadvantages, titanium dioxide is not used in this composition. The desired stability and dissolution profile were achieved with the other excipients (especially colorant) used.

According to one embodiment of the present invention, an oral pharmaceutical composition comprises roxadustat and at least one pharmaceutically acceptable excipient wherein the composition is free of titanium oxide.

According to one embodiment of the present invention, the oral pharmaceutical composition is the film coated tablet comprising core and a film coating.

According to one embodiment of the present invention, at least one colorant is present in the tablet core and/or in the coating. Preferably, at least one colorant is present in the coating.

According to one embodiment of the present invention, the oral pharmaceutical composition is a capsule and at least one colorant is present in the capsule fill and/or in the capsule shell.

Suitable colorants are selected from the group comprising organic dyes and their Lakes, inorganic colors (Pigments), natural colors or mixtures thereof.

According to one embodiment of the present invention, the colorant is selected from organic dyes and their lakes group which comprises sunset Yellow, tartrazine, erythrosine, indigo Carmins, Lakes (Allura Red Aliminium Lake) or mixtures thereof.

According to one embodiment of the present invention, the colorant is selected from natural colors group which comprises riboflavin, carmine, anthocyanins or mixtures thereof.

According to one embodiment of the present invention, the colorant is selected from inorganic dyes group which comprises calcium carbonate, isomalt, iron oxides (Iron Oxide Yellow / Iron Oxide Red / Iron Oxide Black), talc or mixtures thereof. Preferably, at the composition, inorganic dyes are used as colorant. We found that when inorganic dyes (pigments) are used as colorants, it provides long shelf-life, stable throughout the shelf life, ease of processing by drying quickly during the process stages, also it prevents sticking.

According to one embodiment of the present invention, the oral pharmaceutical composition is free of titanium oxide and the composition comprising;
- A core tablet or capsule fill comprising Roxadustat
- A film coating or capsule shell comprising at least one colorant which is selected from inorganic dyes group. It provides the composition that prevent or reduce the amount of photodegradation of Roxadustat. So, desired stability is provided.

According to one embodiment of the present invention, the amount of colorants is between 1.0 % and 30.0% by weight of the film coating or the capsule shell.

According to one embodiment of the present invention, the oral pharmaceutical composition is free of titanium oxide and the composition is a film coating tablet which comprising;
- A core tablet comprising Roxadustat
- A film coating comprising at least one colorant which is selected from inorganic dyes group.

According to one embodiment of the present invention, the amount of colorants is between 1.0 % and 30.0% by weight of the film coating.

According to one embodiment of the present invention, at a film coating tablet, the coating further comprises at least one polymer or at least one plasticizer or at least one absorption enhancer or mixtures thereof.

Suitable polymers are selected from the group comprising hydroxypropyl methylcellulose, hydroxypropyl cellulose, hydroxyethyl cellulose, carboxymethyl cellulose sodium, polyvinylpyrrolidone, polyvinyl alcohol, polyvinyl acetate copolymers, polyvinyl alcohol, polyethylene glycol copolymers, polyethylene glycol, amino-alkyl methacrylate copolymers, maltodextrins, polysaccharides, lactose, polydextrose, isomalt or mixtures thereof.

According to one embodiment of the present invention, the polymer is polyvinyl alcohol, isomalt, polysaccharides (Pullulan), hydroxypropyl methylcellulose or mixtures thereof.

Suitable plasticizers are selected from the group comprising propylene glycol, glycerol, polyethylene glycols, glyceryl triacetate, triethyl citrate, acetyl triethyl citrate, diethyl phthalate, acetylated monoglycerides, castor oil, stearic acid, mineral oil or mixtures thereof.

Suitable absorption enhancers are selected from the group comprising sodium salicylate, medium/long-chain glycerides, taurodihydro fusidate, sodium taurocholate, sodium taurodeoxycholate, lecithin (phosphatidylcholine), sodium dodecyl sulfate, sodium dioctyl sulfosuccinate, EDTA, EGTA, toxins and venom extracts, poly (acrylic acid) derivatives, chitosan salts, n-trimethyl chitosan chloride or mixtures thereof.

According to one embodiment of the present invention, the absorption enhancer is lecithin. The presence of lecithin in the film coating provides dissolution and penetration properties, thus improving bioavailability.

According to one embodiment of the present invention, the oral pharmaceutical composition is free of titanium oxide and the composition is a film coating tablet, the coating comprises;
- at least one colorant which is selected from the inorganic dyes group,
- At least one polymer,
- Lecithin as an absorption enhancer,
- At least one plasticizer.

According to one embodiment of the present invention, the amount of roxadustat is between 10.0% and 45.0% by weight of the total composition.

According to one embodiment of the present invention, the amount of roxadustat is between 20.0% and 40.0% by weight of the total composition.

According to one embodiment of the present invention, the oral pharmaceutical composition comprises at least one pharmaceutically acceptable excipient which is selected from the group comprising fillers, binders, disintegrants, lubricants or mixtures thereof.

Suitable fillers are selected from the group comprising microcrystalline cellulose, lactose monohydrate, ammonium alginate, calcium carbonate, calcium phosphate, calcium phosphate dehydrate, neutral pellets, calcium sulfate, cellulose, cellulose acetate, compressible sugar, ethylcellulose, fructose, glyceryl palmitostearate, lactitol, lactose, mannitol, magnesium carbonate, magnesium oxide, maltodextrin, maltose, medium chain triglycerides, polydextrose, polymethacrylates, sodium alginate, sodium chloride, sugar spheres, sulfobutylether betacyclodextrin, talc, tragacanth, trehalose, xylitol or mixtures thereof.

According to one embodiment of the present invention, the filler is microcrystalline cellulose or lactose monohydrate or mixtures thereof.

According to one embodiment of the present invention, the amount of fillers is between 35.0% and 85.0% by weight of the total composition.

Suitable binders are selected from the group comprising polyvinylpyrrolidone, pregelatinized starch, sugars, natural gums, agar, alginates, carbomers, carboxymethylcellulose sodium, cellulose acetate phthalate, chitosan, starch, corn starch, dextrin, dextrose, ethylcellulose, glyceryl behenate, hydroxyethyl cellulose, hydroxyethyl methylcellulose, hydroxypropyl cellulose, hydroxypropyl starch, hypromellose, magnesium aluminum silicate, maltodextrin, maltose, methylcellulose, poloxamer, polydextrose, polyethylene oxide, polymethacrylates, sucrose or mixtures thereof.

According to one embodiment of the present invention, the binder is polyvinylpyrrolidone.

According to one embodiment of the present invention, the amount of binders is between 1.0% and 15.0% by weight of the total composition.

According to one embodiment of the present invention, the amount of binders is between 2.0% and 10.0% by weight of the total composition.

Suitable disintegrants are selected from the group comprising croscarmellose sodium, low-substituted hydroxypropyl cellulose, sodium carboxymethyl cellulose, calcium carboxymethyl cellulose, carboxymethyl cellulose, docusate sodium, polyacrylate potassium, sodium alginate, alginic acid, magnesium aluminum silica, poloxamer, sodium glycine carbonate or mixtures thereof.

According to one embodiment of the present invention, the disintegrant is croscarmellose sodium.

According to one embodiment of the present invention, the amount of disintegrant is between 1.0% and 15.0% by weight of the total composition.

According to one embodiment of the present invention, the amount of disintegrant is between 2.0% and 10.0% by weight of the total composition.

Suitable lubricants are selected from the group comprising magnesium stearate, silica colloidal anhydrous, sodium stearyl fumarate, talc, polyethylene glycol, aluminum silicate or mixtures thereof.

According to one embodiment of the present invention, the lubricant is magnesium stearate.

According to one embodiment of the present invention, the amount of lubricants is between 0.5% and 8.0% by weight of the total composition.

According to one embodiment of the present invention, the oral pharmaceutical composition of the present invention can be prepared, using standard techniques and manufacturing processes well known in the art, such as direct compression, wet or dry granulation, hot melt granulation, hot melt extrusion, fluidized bed granulation, extrusion/spheronization, slugging, spray drying and solvent evaporation.

According to one embodiment of the present invention, the composition is obtained by using a wet granulation method therefore, a simple and low-cost production method was employed. Wet granulation process efficiently counteracts segregation, so it can achieve good dissolution and disintegration properties. Solvents are used in the process.

The solvent is selected from the group comprising water, acetone, ethanol, isopropanol or mixtures thereof. Preferably, water is used in the present invention.

### Example 1: A film coated tablet

| **Ingredients** | | **Amount (% by weight of the total formulation)** |
|---|---|---|
| Roxadustat | | 10.0 -45.0 |
| Lactose monohydrate | | 30.0 -60.0 |
| Microcrystalline cellulose | | 5.0 - 30.0 |
| Croscarmellose sodium | | 1.0-15.0 |
| Povidone(PVP) | | 1.0 -15.0 |
| Magnesium stearate | | 0.5 -8.0 |
| **TOTAL CORE TABLET** | | **100** |
| **Coating Material** | | |
| | - Polymer / Polymers (15-90%) | |
| | - Plasticizer (0-50%) | |
| | - Colorants (1-30%) | |
| | - Absorption Enhancer (0-50%) | |
| **TOTAL FILM COATED TABLET WEIGHT** | | |

### Example 2: A film coated tablet

| **Ingredients** | | **Amount (% by weight of the total formulation)** |
|---|---|---|
| Roxadustat | | 20.0 -40.0 |
| Lactose monohydrate | | 40.0 -58.0 |
| Microcrystalline cellulose | | 8.0 - 20.0 |
| Croscarmellose sodium | | 2.0-10.0 |
| Povidone(PVP) | | 2.0 -10.0 |
| Magnesium stearate | | 0.5 -5.0 |
| **TOTAL CORE TABLET** | | **100** |
| **Coating Material** | | |
| | - Polymer / Polymers (20-80%) | |
| | - Plasticizer (1-40%) | |
| | - Colorants (1-30%) | |
| | - Absorption Enhancer (1-40%) | |
| **TOTAL FILM COATED TABLET WEIGHT** | | |

### Example 3: A film coated tablet

| **Ingredients** | | **Amount (% by weight of the total formulation)** |
|---|---|---|
| Roxadustat | | 25.0 |
| Lactose monohydrate | | 50.6 |
| Microcrystalline cellulose | | 12.5 |
| Croscarmellose sodium | | 5.3 |
| Povidone (PVP) | | 5.3 |
| Magnesium stearate | | 1.3 |
| **TOTAL CORE TABLET** | | **100** |
| **Coating Material** | | |
| | - Polymer / Polymers (15-90%) | |
| | - Plasticizer (0-50%) | |
| | - Colorants (1-30%) | |
| | - Absorption Enhancer (0-50%) | |
| **TOTAL FILM COATED TABLET WEIGHT** | | |

A process for example 1 or 2 or 3;
a) Mixing roxadustat, microcrystalline cellulose and lactose monohydrate and croscarmellose sodium,
b) Dissolving povidone in pure water,
c) Granulating the mixture at step (a) with the solution at step (b),
d) Drying the wet granules and sieving,
e) Adding magnesium stearate and then mixing,
f) Compressing the mixture into the tablet,
g) Coating the tablets with film coating agent.

## Claims

1. An oral pharmaceutical composition comprises roxadustat and at least one pharmaceutically acceptable excipient wherein the composition is free of titanium oxide.

2. The oral pharmaceutical composition according to claim 1, wherein the composition is the film coated tablet comprising core and a film coating.

3. The oral pharmaceutical composition according to claim 2, wherein at least one colorant is present in the tablet core and/or in the coating.

4. The oral pharmaceutical composition according to claim 1, wherein the composition is a capsule and at least one colorant is present in the capsule fill and/or in the capsule shell.

5. The oral pharmaceutical composition according to claim 3 or 4, wherein colorants are selected from the group comprising organic dyes and their Lakes, inorganic colors (Pigments), natural colors or mixtures thereof.

6. The oral pharmaceutical composition according to claim 5, wherein the colorant is selected from inorganic dyes group which comprises calcium carbonate, Isomalt, iron oxides (Iron Oxide Yellow / Iron Oxide Red / Iron Oxide Black), talc or mixtures thereof.

7. The oral pharmaceutical composition according to claim 6, wherein the composition comprising;
- A core tablet or capsule fill comprising Roxadustat
- A film coating or capsule shell comprising at least one colorant which is selected from inorganic dyes group.

8. The oral pharmaceutical composition according to claim 7, wherein the amount of colorants is between 1.0 % and 30.0% by weight of the film coating or the capsule shell.

9. The oral pharmaceutical composition according to claim 2, wherein, at a film coating tablet, the coating further comprises at least one polymer or at least one plasticizer or at least one absorption enhancer or mixtures thereof.

10. The oral pharmaceutical composition according to claim 9, wherein polymers are selected from the group comprising hydroxypropyl methylcellulose, hydroxypropyl cellulose, hydroxyethyl cellulose, carboxymethyl cellulose sodium, polyvinylpyrrolidone, polyvinyl alcohol, polyvinyl acetate copolymers, polyvinyl alcohol, polyethylene glycol copolymers, polyethylene glycol, amino-alkyl methacrylate copolymers, maltodextrins, polysaccharides, lactose, polydextrose, isomalt or mixtures thereof.

11. The oral pharmaceutical composition according to claim 9, wherein the polymer is polyvinyl alcohol, isomalt, polysaccharides (Pullulan), hydroxypropyl methylcellulose or mixtures thereof.

12. The oral pharmaceutical composition according to claim 9, wherein plasticizers are selected from the group comprising propylene glycol, glycerol, polyethylene glycols, glyceryl triacetate, triethyl citrate, acetyl triethyl citrate, diethyl phthalate, acetylated monoglycerides, castor oil, stearic acid, mineral oil or mixtures thereof.

13. The oral pharmaceutical composition according to claim 9, wherein absorption enhancers are selected from the group comprising sodium salicylate, medium/long-chain glycerides, taurodihydro fusidate, sodium taurocholate, sodium taurodeoxycholate, lecithin (phosphatidylcholine), sodium dodecyl sulfate, sodium dioctyl sulfosuccinate, EDTA, EGTA, toxins and venom extracts, poly (acrylic acid) derivatives, chitosan salts, n-trimethyl chitosan chloride or mixtures thereof.

14. The oral pharmaceutical composition according to claim 13, wherein the absorption enhancer is lecithin.

15. The oral pharmaceutical composition according to claim 2, wherein the coating comprises;
- at least one colorant which is selected from the inorganic dyes group,
- At least one polymer,
- Lecithin as an absorption enhancer,
- At least one plasticizer.
